# EUROPEAN PATENT APPLICATION

(11) **EP 3 970 700 A1**
(43) Date of publication of application: **23.03.2022**
(21) Application number: 21197089.2
(22) Date of filing: 16.09.2021
(51) Int. Cl.: A61K 9/16, A61K 9/20, A61K 9/48, A61K 31/4152

(54) **A SOLID ORAL PHARMACEUTICAL FORMULATION COMPRISING ELTROMBOPAG OLAMINE**

(30) Priority: 16.09.2020 TR 202014694
(71) Applicant: Sanovel Ilac Sanayi ve Ticaret A.S., 34460 Sariyer/Istanbul (TR)
(72) Inventor: SUNEL, Fatih, 34460 Istanbul (TR); TOK, Gulcin, 34460 Istanbul (TR)
(74) Representative: Sevinç, Erkan

(57) **Abstract**

The present invention relates to a solid oral pharmaceutical formulation comprising the particle size d(0.9) of the eltrombopag olamine is less than 10 µm. The formulation eliminates problems of the active agent and provide additional advantages to the relevant prior art.

## Description

### Field of the Invention

The present invention relates to a solid oral pharmaceutical formulation comprising the particle size d(0.9) of the eltrombopag olamine is less than 10 µm. The formulation eliminates problems of the active agent and provide additional advantages to the relevant prior art.

### Background of the Invention

Thrombopoietin (THPO), also known as megakaryocyte growth and development factor (MGDF), is a protein that in humans is encoded by the THPO gene.

The thrombopoietin receptor agonists mimic the action of thrombopoietin on its receptor and stimulate the activation, proliferation and maturation of megakaryocytes, resulting in an increase in circulating platelet counts. Thrombopoietin itself acts in this manner, but when recombinant thrombopoietins were used clinically, they were found to cause rebound thrombocytopenia, probably due to induction of anti-thrombopoietin antibodies. For this reason, direct administration of thrombopoietin was abandoned as an approach to treating thrombocytopenia and other approaches for activating the thrombopoietin receptor were sought.

Two thrombopoietin receptor agonists were subsequently developed and are now in clinical use for chronic idiopathic thrombocytopenic purpura (ITP) and for other thrombocytopenic conditions.

Eltrombopag olamine is a peptide-like, small molecular weight agonist of the thrombopoietin receptor. This agent is given by mouth and result in significant increases in platelet counts in normal persons as well as patients with idiopathic thrombocytopenic purpura (ITP).

Eltrombopag olamine is a small molecular weight peptide-like molecule that binds to the transmembrane domain of the thrombopoietin receptor and causes its activation and the proliferation and differentiation of megakaryocytes, with a resultant increase in synthesis and release of platelets. In multiple clinical trials, eltrombopag olamine was shown to raise the platelet count in patients with idiopathic thrombocytopenic purpura (ITP), aplastic anemia and cirrhosis due to chronic hepatitis C during interferon therapy. Eltrombopag olamine was approved for use in the United States in 2008 for the treatment of ITP and its indications have subsequently been expanded to other thrombocytopenic conditions.

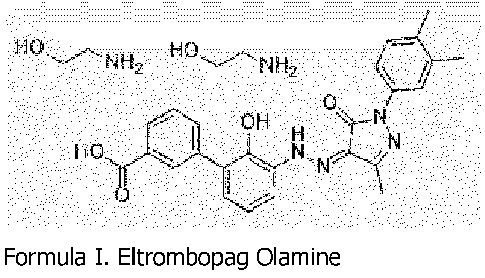

In the state of art, the application EP1889838B1 is the molecule patent of eltrombopag olamine. Another application EP3041511A2 discloses pharmaceutical compounds of eltrombopag olamine in the form of tablets and the methods for their preparation.

Eltrombopag olamine presents the formulator with unique concerns when attempting to formulate this compound into a suitable solid oral pharmaceutical dosage form, suitably a tablet, suitably a capsule, with a desirable pharmacokinetic profile. Some of the concerns is that slow dissolution of the compound from solid dosage forms, the tendency of the compound to form insoluble metal complexes when contacted with excipients that contain a coordinating metal, and the tendency of the compound to under-go a Maillard reaction when contacted with excipients that contain reducing sugars. In other words, eltrombopag olamine is very sensitive for some excipients and environmental effects.

EP3615007 (A1) discloses a pharmaceutical tablet composition comprising eltrombopag olamine and one or more reducing sugars, a production process therefore, a pharmaceutical tablet composition comprising eltrombopag olamine and one or more reducing sugars obtainable by the production process.

WO03/098992 (A2) discloses an eltrombopag tablet composition comprising 8.45 mg eltrombopag olamine, furthermore 112 mg microcrystallinecellulose, 70 mg lactose, 8 mg sodium starch glycolate and 2 mg magnesium stearate.

In this sense, there is still a need for an eltrombopag olamine formulation in the solid form which eliminates the above described problem of eltrombopag olamine and which enhances the dissolution profile and bioavailability of the final pharmaceutical formulation.

In the present invention, it is found that the use of micronized eltrombopag olamine provided great advantages in the dissolution profile and hence bioavailability. Considering the above described disadvantages of the active ingredient, a unique formulation has been created with compatible excipients and micronized eltromopag olamine.

### Detailed Description of the Invention

The main object of the present invention is to provides a solid oral pharmaceutical formulation comprising eltrombopag olamine having desired dissolution profile, stability (chemical stability or towards dehydration and/or storage stability) for the treatment of conditions leading to thrombocytopenia.

According to an embodiment of the present invention, in the solid oral pharmaceutical formulation comprising the eltrombopag olamine, the particle size seems to play an active role in increasing the solubility of the active substance, eltrombopag olamine, and providing an appropriate bioavailability.

The term "particle size" refers to the cumulative volume size distribution tested by any conventionally recognised method, such as a laser diffraction method. The term d (0.9) means the size at which 90% by volume of the particles are finer.

Particularly, the particle size distribution of D (0.9) overcome the problems encountered in the prior art by increasing the solubility of eltrombopag olamine, resulting in enhanced bioavailability of the solid oral formulation.

According to an embodiment of the present invention, a solid oral pharmaceutical formulation comprises eltrombopag olamine and at least one pharmaceutically acceptable excipient, wherein the particle size d (0.9) of the eltrombopag olamine is less than 10 µm. The particle size helps to ensure the desired dissolution profile.

According to an embodiment of the present invention, the particle size d (0.9) of eltrombopag olamine is preferably between 9 µm and 1 µm or between 8 µm and 2 µm, between 7 µm and 3 µm, between 6 µm and 4 µm.

According to an embodiment of the present invention, the amount of eltrombopag olamine is between 5.0% and 30.0% by weight in the total formulation.

According to an embodiment of the present invention, the amount of eltrombopag olamine is between 7.0% and 28.0% by weight in the total formulation.

According to one embodiment of the present invention, the formulation comprises at least one pharmaceutical acceptable excipient which are selected from binders, diluents, disintegrants, glidants, lubricants or mixtures thereof.

Suitable binders are selected from the group comprising polyvinylpyrrolidone (povidone), natural gums, agar, alginates, carbomers, carboxymethylcellulose sodium, cellulose acetate phthalate, copovidone, starch, starch mucilage, dextrates, ethylcellulose, glyceryl behenate, guar gum, methylcellulose, poloxamer, polycarbophil, polyethylene oxide, polymethacrylates, stearic acid, cetostearyl alcohol, polyoxyethilene-alkyl ethers, pullulan or mixtures thereof.

According to one embodiment of the present invention, the amount of binders is between 0.1% and 5.0% in the total formulation.

According to one embodiment of the present invention, the binder is polyvinylpyrrolidone. Using polyvinylpyrrolidone helps to provide homogeneous formulation, even when used in small amounts.

Suitable diluents are selected from the group comprising microcrystalline cellulose, mannitol, spray-dried mannitol, sucrose, sorbitol, xylitol, inositol, inorganic salts, calcium salts, polysaccharides, dicalcium phosphate, sodium chloride, dextrates, lactitol, maltodextrin, sucrose-maltodextrin mixture, sodium carbonate, sodium bicarbonate, calcium carbonate or mixtures thereof.

According to one embodiment of the present invention, the amount of diluents is between 30.0% and 85.0% in the total formulation.

According to one embodiment of the present invention, the diluents is microcrystalline cellulose or mannitol or mixtures thereof. In the current state of art, the use of mannitol or microcrystalline cellulose contributes to maintaining the stability in the formulation, particularly by balancing the moisture-sensitive active ingredient eltrombopag olamine with its non-hygroscopic property.

Suitable disintegrants are selected from the group comprising croscarmellose sodium, sodium starch glycolate, alginic acid, alginates, ion-exchange resins, sodium carboxymethyl cellulose, carboxymethyl cellulose, calcium carboxymethyl cellulose, docusate sodium, low substituted hydroxypropyl cellulose, polyacryline potassium, poloxamer, sodium alginate, sodium glycine carbonate, sodium dodecyl sulfate or mixtures thereof.

According to one embodiment of the present invention, the amount of disintegrants is between 2.0% and 20.0% in the total formulation.

According to one embodiment of the present invention, the disintegrant is croscarmellose sodium.

Suitable glidants are selected from the group comprising talc, colloidal silica, magnesium silicate, magnesium oxide, starch or mixtures thereof.

According to one embodiment of the present invention, the amount of glidants is between 0.1% and 3.0% in the total formulation.

According to one embodiment of the present invention, the glidant is colloidal silicon dioxide.

Suitable lubricants are selected from group comprising sodium stearyl fumarate, magnesium stearate, stearic acid, calcium stearate, zinc stearate, hydrogenated castor oil, hydrogenated vegetable oil, light mineral oil, mineral oil, sodium benzoate or mixtures thereof.

According to one embodiment of the present invention, the amount of lubricants is between 1.0% and 5.0% in the total formulation.

According to one embodiment of the present invention, the lubricant is sodium stearyl fumarate.

According to one embodiment of the present invention, the formulation comprises eltrombopag olamine, microcrystalline cellulose, mannitol, polyvinylpyrrolidone (povidon k-30), croscarmellose sodium, colloidal silicon dioxide, sodium stearyl fumarate.

According to one embodiment of the present invention, the solid oral pharmaceutical formulation is in the form of tablets, capsules, strips, pastilles, sachets.

According to one embodiment of the present invention, the solid oral pharmaceutical formulation is in the form of capsule.

According to one embodiment of the present invention, the solid oral pharmaceutical formulation is in the form of tablet, preferably film-coated tablet, which may vary in shape and be, e.g., round, oval, oblong, cylindrical, caplet shaped or any other suitable shape, preferably it is round or caplet shaped.

According to one embodiment of the present invention, process of preparing the composition is wet granulation, dry granulation, hot melt extrusion, direct compression, spray drying or mixtures thereof.

According to one embodiment of the present invention, the solid oral pharmaceutical formulation is obtained by using a wet granulation method.

According to one embodiment of the present invention, the solid oral pharmaceutical formulation is for use in preventing or treating conditions leading to thrombocytopenia.

### Example 1: The capsule or tablet formulation comprising Eltrombopag Olamine

| Ingredients | % by weight |
|---|---|
| Eltrombopag Olamine | 5.0- 30 |
| Microcrystalline cellulose | 25.0 - 75.0 |
| Mannitol | 5.0 - 27.0 |
| Polyvinylpyrrolidone (Povidon K-30) | 0.1 - 5.0 |
| Croscarmellose Sodium | 2.0 - 20.0 |
| Colloidal silicon dioxide | 0.1 - 3.0 |
| Sodium stearyl fumarate | 1.0 - 5.0 |
| TOTAL | 100 |

### Example 2: The capsule or tablet formulation comprising Eltrombopag Olamine

| | Formulation 1 | Formulation 2 | Formulation 3 |
|---|---|---|---|
| Eltrombopag Olamine | 15.95 mg | 63.8 mg | 95.7 mg |
| Microcrystalline cellulose | 130.94 mg | 180.37 mg | 98.85 mg |
| Mannitol | 14.875 mg | 59.5 mg | 89.25 mg |
| Polyvinylpyrrolidone (Povidon K-30) | 1.6 mg | 6.4 mg | 9.6 mg |
| Croscarmellose Sodium | 8.335 mg | 33.33 mg | 50 mg |
| Colloidal silicon dioxide | 1.75 mg | 3.5 mg | 3.5 mg |
| Sodium stearyl fumarate | 4.05 mg | 8.1 mg | 8.1 mg |
| Coating (For tablet) | 5.325 mg | 10.65 mg | 10.65 mg |
| Total weight | 182.825 | 365.65 | 365.65 |

Process for example 1 or 2;
a) Mixing eltrombopag olamine, microcrystalline cellulose, mannitol, polyvinylpyrrolidone,
b) Granulating the mixture with pure water in a high-shear wet-granulator,
c) Sieving the mixture,
d) Drying the mixture in the fluid bed dryer,
e) Sieving the mixture and obtained homogenous powder,
f) Then, mixing microcrystalline cellulose, croscarmellose sodium and colloidal silicon dioxide and adding to the mixture from step (e),
g) Adding sodium stearyl fumarate and mixing,
h) For capsule; Filling the powder mixture into the hard gelatin capsule or

For tablet; Compressing to form of tablets and coating tablets with film coating.

## Claims

1. A solid oral pharmaceutical formulation comprising eltrombopag olamine and at least one pharmaceutically acceptable excipient, wherein the particle size (0.9) of the eltrombopag olamine is less than 10 µm.

2. The solid oral pharmaceutical formulation according to claim 1, wherein the particle size d (0.9) of eltrombopag olamine is preferably between 9 µm and 1 µm or between 8 µm and 2 µm, between 7 µm and 3 µm, between 6 µm and 4 µm.

3. The solid oral pharmaceutical formulation according to claim 1, wherein the amount of eltrombopag olamine is between 5.0% and 30.0% by weight in the total formulation.

4. The solid oral pharmaceutical formulation according to claim 1, wherein the formulation comprising at least one pharmaceutical acceptable excipient which are selected from binders, diluents, disintegrants, glidants, lubricants or mixtures thereof.

5. The solid oral pharmaceutical formulation according to claim 4, wherein binders are selected from the group comprising polyvinylpyrrolidone (povidone), natural gums, agar, alginates, carbomers, carboxymethylcellulose sodium, cellulose acetate phthalate, copovidone, starch, starch mucilage, dextrates, ethylcellulose, glyceryl behenate, guar gum, methylcellulose, poloxamer, polycarbophil, polyethylene oxide, polymethacrylates, stearic acid, cetostearyl alcohol, polyoxyethilene-alkyl ethers, pullulan or mixtures thereof.

6. The solid oral pharmaceutical formulation according to claim 5, wherein the amount of binders is between 0.1% and 5.0% in the total formulation.

7. The solid oral pharmaceutical formulation according to claim 5, wherein the binder is polyvinylpyrrolidone.

8. The solid oral pharmaceutical formulation according to claim 4, wherein diluents are selected from the group comprising microcrystalline cellulose, mannitol, spray-dried mannitol, sucrose, sorbitol, xylitol, inositol, inorganic salts, calcium salts, polysaccharides, dicalcium phosphate, sodium chloride, dextrates, lactitol, maltodextrin, sucrose-maltodextrin mixture, sodium carbonate, sodium bicarbonate, calcium carbonate or mixtures thereof.

9. The solid oral pharmaceutical formulation according to claim 8, wherein the amount of diluents is between 30.0% and 85.0% in the total formulation.

10. The solid oral pharmaceutical formulation according to claim 8, wherein the diluent is microcrystalline cellulose or mannitol or mixtures thereof.

11. The solid oral pharmaceutical formulation according to claim 4, wherein the formulation comprising eltrombopag olamine, microcrystalline cellulose, mannitol, polyvinylpyrrolidone (povidon k-30), croscarmellose sodium, colloidal silicon dioxide, sodium stearyl fumarate.

12. The solid oral pharmaceutical formulation according to claim 1, wherein the solid oral pharmaceutical formulation is in the form of tablets, capsules, strips, pastilles, sachets.

13. The solid oral pharmaceutical formulation according to claim 12, wherein the solid oral pharmaceutical formulation is in the form of capsule.

14. The solid oral pharmaceutical formulation according to claim 12, wherein the solid oral pharmaceutical formulation is in the form of tablet.

15. The solid oral pharmaceutical formulation according to claim 1, wherein the formulation is obtained by using a wet granulation method.
